# EUROPEAN PATENT SPECIFICATION

(11) **EP 2 509 622 B1**
(45) Date of publication and mention of the grant of the patent: **20.08.2014**
(21) Application number: 10836535.4
(22) Date of filing: 07.12.2010
(51) Int. Cl.: A61K 38/44, A61K 38/54, A61K 38/45, A61K 33/38, A61K 33/30, A61K 9/06, A61K 9/70, A61P 31/04

(54) **INHIBITING BACTERIAL INFECTION AND BIOFILM FORMATION**
HEMMUNG DER BAKTERIENINFEKTION UND BIOFILMBILDUNG
INHIBITION DES INFECTIONS BACTÉRIENNES ET DE LA FORMATION DE BIOFILMS

(30) Priority: 09.12.2009 US 285009 P
(43) Date of publication of application: 17.10.2012
(73) Proprietor: KCI Licensing, Inc., San Antonio, TX 78265 (US)
(72) Inventor: MILLER, Cynthia, San Antonio TX 78265 (US); MCNULTY, Amy, San Antonio TX 78265 (US); JOHNSON, Royce, San Antonio TX 78265 (US)
(74) Representative: Cordina, Kevin John
(86) International application number: PCT/US2010/059261
(87) International publication number: WO 2011/071904

(56) References cited:
- EP-A1- 0 277 383
- WO-A1-00/78141
- WO-A1-2004/063254
- WO-A1-2005/055723
- US-A- 5 500 448
- US-A1- 2006 289 354
- US-A1- 2007 003 538
- US-A1- 2007 202 138
- ARCH PERSONAL CARE PRODUCTS: "Product Data Biovert Enzyme and Substrate", INTERNET CITATION, 6 November 2007 (2007-11-06), pages 1-4, XP002485794, Retrieved from the Internet: URL:http://www.archchemicals.com/Fed/PC/Do cs/Biovert_v2.1.pdf [retrieved on 2008-06-25]

## Description

### BACKGROUND OF THE INVENTION

This application claims priority to United States Provisional Patent Application Serial No. 61/285,009, filed on December 9, 2009.

### 1. Field of the Invention

The present invention relates generally to the field of treating bacterial infections. More particularly, it relates to antimicrobial agents and their use in eliminating biofilm and planktonic cells.

### 2. Description of the Related Art

Biofilm formation and planktonic proliferation by undesired microorganisms are well known phenomena in domestic as well as industrial settings. For example, it is believed that all wounds are colonized by microbes. If the microbes reach a level of clinical infection, their presence is believed to impair healing and may be a contributing factor to wound chronicity. Recently researchers have proposed that it may not be planktonic but rather biofilm communities which contribute to wound chronicity.

Biofilms are polymicrobial groupings of bacteria which are held together in an extracellular polymeric substance consisting of protein, DNA, and polysaccarhides and are not totally susceptible to antibiotic treatment. In fact, recent research (James *et al.,* 2008) has shown that 60% of the chronic wounds tested contained biofilm. Therefore, one of the most important aspects of wound treatment is the concept of controlling bioburden, or the microbial levels during processing and handling.

EP0277383 discloses mouth-care products containing an oxidoreductase and a combination of an 8-hydroxyquinoline and a compound yielding zinc ions.

The product data sheet for the Biovert® Enzyme and Substrate System discloses the use of glucose oxidase, lactoperoxidase and glucose in an antimicrobial system.

### SUMMARY OF THE INVENTION

The present invention is as set out in the claims.

The present invention relates generally to the field of treating bacterial infections. More particularly, it relates to antimicrobial agents and their use in eliminating biofilm and planktonic cells.

The present invention provides an antimicrobial agent formulation comprising a natural enzyme and substrate system comprising lactoperoxidase, glucose oxidase, and glucose; an antimicrobial metal; and a zwitterionic detergent. In certain embodiments, one or more of the agents in the formulation are encapsulated. In one embodiment, the encapsulating agent is a multi-layered microsphere of surfactants. In other embodiments, the agents in the formulation are not encapsulated.

The enzyme and substrate components may be present in any suitable ratio, as would be recognized by a person having skill in the art. In some embodiments, the enzyme and substrate components may be present in a 1:1, 1:5, 1:10, 1:15, 1:20, 1:25, 1:30, and/or 1:40 ratio, or any ratio derivable in between. In particular embodiments, the enzyme and substrate components are present in a 1:20 ratio. In the present invention, the enzyme and substrate components combine to form 1% to 5% (v/v) of the final formulation. In particular embodiments, the enzyme and substrate components combine to form 1.25% to 2% (v/v) of the final formulation.

The antimicrobial metal is gallium. Copper, zinc, or silver are also antimicrobial metals disclosed herein. In some embodiments, the antimicrobial agent contains two or more antimicrobial metals. In still other embodiments these metals are present in an oxide form or in organically available forms, such as silver oxide or silver taurate.

The detergent is a zwitterionic detergent selected from lauramine oxide or decylamine oxide.

The antimicrobial agent formulation may be in any formulation known to those having skill in the art. In some embodiments, the formulation is an emulsion, spray, cream, lotion, ointment, hydrogel, or electroporation device cartridge. In particular embodiments, the antimicrobial agent is an a hydrophilic solution. The antimicrobial agent formulation may further comprise additional ingredients known to those having skill in the art. In some embodiments, the additional ingredient may be an antioxidant, a buffering system, a mild surfactant, or a pharmaceutical ingredient.

Herein disclosed is a method of eliminating microorganisms in a biofilm comprising contacting the biofilm with an antimicrobial agent comprising a natural enzyme and substrate system comprising lactoperoxidase, glucose oxidase, and glucose; an antimicrobial metal; and a zwitterionic detergent.

The biofilm may be located on a patient or a surface, such as a surgical instrument, infected hardware, or an implanted device. In some embodiments, the patient is a human patient. In some embodiments, the patient may have an injury. In particular embodiments, the injury may be a bum, abrasion, cut, scrape, denuding tissue injury or combinations thereof. In other embodiments, the patient may be afflicted with a chronic wound. In particular embodiments, the chronic wound is a venous ulcer, diabetic ulcer, arterial ulcer, pressure ulcer, radiation ulcer, traumatic wound, non-healing wound or combinations thereof.

The biofilm may be contacted by the antimicrobial agent in any suitable manner. In some embodiments, contacting the biofilm comprises applying the antimicrobial agent to a wound. In some embodiments, contacting the biofilm comprises administering the composition topically. In particular embodiments, administering the composition topically is selected from administering by hand, administering by an extruder, spray delivery, applying a dressing including the composition, and combinations thereof. In other embodiments, contacting the biofilm comprises applying the composition to a dressing prior to applying the dressing to the patient. In still other embodiments, the antimicrobial agent is contacted topically, intravenously, intradermally, intraarterially, intraperitoneally, intralesionally, intracranially, intraarticularly, intraprostaticaly, intrapleurally, intratracheally, intraocularly, intranasally, intravitreally, intravaginally, intrarectally, intramuscularly, intraperitoneally, subcutaneously, subconjunctival, intravesicularlly, mucosally, intrapericardially, intraumbilically, intraocularally, orally, by inhalation, by injection, by infusion, by continuous infusion, by localized perfusion bathing target cells directly, *via* a catheter, or *via* a lavage. In some embodiments, the biofilm is contacted with the antimicrobial agent two or more times.

The biofilm may be formed by any bacteria capable of forming biofilms. Such bacteria are known to those of skill in the art. In some embodiments, the biofilm is formed by *Pseudomonas aeruginosa, Streptococcus mutants, Streptococcus sanguis, Legionella, Neisseria gonorrhoeae, Staphylococcus aureus* or *Enterococcus sp.* bacteria. In particular embodiments, the biofilm is formed by a *Pseudomorcas* or *Staphylococcus* bacteria.

Herein disclosed is a method of eliminating biofilm-forming microorganisms comprising contacting the biofilm-forming microoganisms with an antimicrobial agent comprising a natural enzyme and substrate system comprising lactoperoxidase, glucose oxidase, and glucose; an antimicrobial metal; and a zwitterionic detergent. In some embodiments, the biofilm-forming microorganisms are in a planktonic state.

The embodiments in the Example section are understood to be embodiments of the invention that are applicable to all aspects of the invention.

The use of the term "or" in the claims is used to mean "and/or" unless explicitly indicated to refer to alternatives only or the alternatives are mutually exclusive, although the disclosure supports a definition that refers to only alternatives and "and/or."

Throughout this application, the term "about" is used to indicate that a value includes the standard devi*a*tion of error for the device or method being employed to determine the value.

Following long-standing patent law, the words "a" and "an," when used in conjunction with the word "comprising" in the claims or specification, denotes one or more, unless specifically noted.

The term "therapeutically effective" as used herein refers to an amount of cells and/or therapeutic composition (such as a therapeutic polynucleotide and/or therapeutic polypeptide) that is employed in methods of the present invention to achieve a therapeutic effect, such as wherein at least one symptom of a condition being treated is at least ameliorated, and/or to the analysis of the processes or materials used in conjunction with these cells.

Other objects, features and advantages of the present invention will become apparent from the following detailed description. It should be understood, however, that the detailed description and the specific examples, while indicating specific embodiments of the invention, are given by way of illustration only, since various changes and modifications within the scope of the invention will become apparent to those skilled in the art from this detailed description.

### BRIEF DESCRIPTION OF THE FIGURES

The following drawings form part of the present specification and are included to further demonstrate certain aspects of the present invention. The invention may be better understood by reference to one or more of these drawings in combination with the detailed description of specific embodiments presented herein.
**FIG. 1****:** A flow chart representing the function of the enzyme substrate system.
**FIG. 2****:** A graph showing the results of a biofilm disruption study on a porcine biofilm model of skin wounds. Control = no treatment; NPWT = treatment with V.A.C.@ Therapy (only) at -125 mmHg; Solution 1 = treatment with lactoperoxidase, glucose oxidase, glucose, lauramine oxide, gallium chloride, Tris HEl; pH 5.3 - 5.9; Solution 2 = treatment with lactoperoxidase, glucose oxidase, and glucose encapsulated in Spherulites™, Lauramine oxide, gallium chloride, Tris HCl, water; pH 5.2 - 5.9.

### DESCRIPTION OF THE ILLUSTRATIVE EMBODIMENTS

The achievements of medical care in industrialized societies are markedly impaired due to chronic infections that have become increasingly apparent in immunocompromised patients and the aging population. Chronic infections remain a major challenge for the medical profession because traditional antibiotic therapy is usually not sufficient to eradicate these infections. One major reason for persistence seems to be the capability of the bacteria to grow within biofilms that protect them from adverse environmental factors. Biofilm is particularly troublesome in acting as a causative factor of chronic wounds because of its persistence and depth within wound-bed tissues.

The current disclosure presents a formulation of an antimicrobial agent system that is effective in eliminating both cells in a biofilm and planktonic cells. Coupling such an antimicrobial agent system with a method to mechanically disrupt the biofilm may further expedite biofilm eradication and wound healing.

### A. Biofilm and Planktonic Cells

A biofilm is an aggregate of microorganisms in which cells are stuck to each other and/or to a surface. In contrast, planktonic cells are single-cells that may float or swim in a liquid medium. The adherent cells found in biofilm are frequently embedded within a self-produced matrix of extracellular polymeric substance, may form on living or non-living surfaces, and represent a prevalent mode of microbial life in natural, industrial and hospital settings. Biofilms form in response to many factors, which may include cellular recognition of specific or non-specific attachment sites on a surface, nutritional cues, or in some cases, by exposure of planktonic cells to sub-inhibitory concentrations of antibiotics. Bacteria cells in a planktonic state may form into a biofilm if left untreated.

Biofilms and planktonic cells are known to be involved in a wide variety of microbial infections in the body. Infectious processes in which biofilms have been implicated include common problems such as urinary tract infections, catheter infections, middle-ear infections, formation of dental plaque, gingivitis, coating contact lenses, endocarditis, and infections in cystic fibrosis. Biofilms can also be formed on the inert surfaces of implanted devices such as catheters, prosthetic cardiac valves and intrauterine devices. Bacterial biofilms may also impair cutaneous wound healing and reduce topical antibacterial efficiency in healing or treating infected skin wounds.

A person having skill in the art would recognize that many bacterias form biofilms. Wolcott *et al.,* 2008 and James *et cal.,* 2008. For example, *Pseudomonas aeruginosa* is known to form biofilms and is an important opportunistic pathogen and causative agent of emerging nosocomial infections. Dental plaque is a biofilm on the surfaces of the teeth and consists of bacterial cells (mainly *Streptococcus mutans* and *Streptococcus sanguis),* salivary polymers and bacterial extracellular products. *Legionella* bacteria are known to grow under certain conditions in biofilms, in which they are protected against disinfectants. *Neisseria gonorrhoeae* is an exclusive human pathogen that has been demonstrated as forming biofilms on glass surfaces and over human cells. Other types of bacteria that form biofilms include *Staphylococcus aureus* and *Enterococcus sp.*

Because of the properties provided by microorganisms in a biofilm, biofilms are typically less susceptible to antibiotics, antimicrobials, and biocides. In some cases, bacteria in a biofilm can be up to 4,000 times more resistant (*i.e*., less susceptible) than the same organism in a planktonic state. Minimum inhibitory concentration (MIC) describes the amount of an active agent delivered to planktonic microorganisms necessary to inhibit biofilm formation. In contrast, minimum biofilm eradication concentration (MBEC) describes the minimum concentration of an active agent delivered to a biofilm necessary to inhibit or eradicate biofilm growth. The differential that can be seen in these amounts illustrates that biofilm-forming microorganisms are much less susceptible to antimicrobial agents at standard therapeutic concentrations.

The current formulation has efficacy against both biofilm and planktonic organisms. The disclosed multi-part antimicrobial agent formulation is also effective against both the persistence and depth within tissues that are characteristic traits of biofilms.

### B. Enzyme Substrate Systems

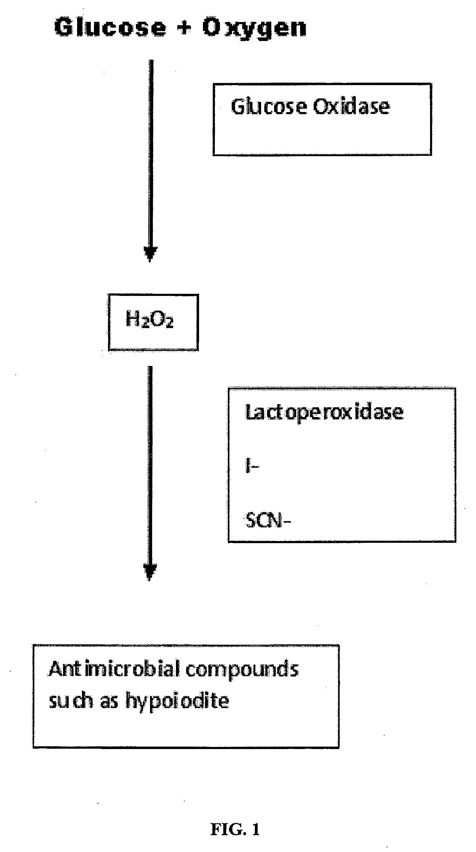

The antimicrobial activity of the present formulation is based on a natural enzyme and substrate system comprising lactoperoxidase, glucose oxidase, and glucose. Lactoperoxidase is a peroxidase enzyme found in milk, and is known to have antimicrobial and antioxidant properties. Glucose (Glc) is a monosaccharide and is a very important carbohydrate in biology. The living cell uses it as a source of energy and metabolic intermediate. Glucose is one of the main products of photosynthesis and starts cellular respiration in both prokaryotes, including bacteria, and eukaryotes. The glucose oxidase enzyme (GOx) binds to beta-D-glucopyranose and aids in breaking the sugar down into its metabolites. Glucose oxidase acts as a natural preservative by reducing atmospheric O₂ to hydrogen peroxide (H₂O₂), which acts as an antimicrobial barrier. In an exemplary embodiment, the enzymatic composition is sold by Arch Personal Care Products, L.P. under the tradename "Biovert Enzyme & Substrate." Without wishing to be bound by any particular theory, it is believed that in the presence of glucose, the glucose oxidase generates hydrogen peroxide. The hydrogen peroxide is then used by lactoperoxidase to form hypoiodite and hypothiocyanate (FIG. 1). Both hypoiodite and hypothiocyanate have good antimicrobial activity that results in the rapid death of the microbial cell.

Within some embodiments of the present invention, the enzyme and substrate components are used at a range of stoichiometric ratios including 1:1, 1:5, 1:10, 1:15, 1:20, 1:25, 1:30, and/or 1:40 ratio, or any ratio derivable in between. In particular embodiments, the enzyme and substrate components are used at a 1:20 ratio. Other sugars as the enzyme substrate, such as sucrose or fructose, are also disclosed herein in which case the ratio of substrate to enzyme will change to match the reaction requirements for the pairing. The enzyme and substrate components combine to form 1% to 5% (v/v) of the final formulation. In particular embodiments, the enzyme and substrate components combine to form 1.25% to 2% (v/v) of the final formulation.

### C. Detergents

The antimicrobial agent formulation further comprises a detergent. Ananthapadmanabhan et al., 2004. The addition of the detergent helps to emulsify components of the biofilm, making them more susceptible to inhibition or damage by the enzyme system. The detergent also aids penetration of the agent into the affected tissues. In some embodiments, the antimicrobial agent formulation comprises a low level of a detergent. A low level of the detergent may be anywhere from 0.25% to 5% (v/v). In particular embodiments, the level of the detergent is 0.25% to 1.5% (v/v). The detergent is a gentle zwitterionic detergent selected from the Generally Regarded As Safe (GRAS) designated detergents lauramine oxide and decylamine oxide.

### D. Antimicrobial Metals

The antimicrobial agent formulation also contains the antimicrobial metal gallium, which synergistically boosts the efficacy of the enzyme system. Metals having known antimicrobial properties are known to those of skill in the art. Michels 2009 and Kaneko 2007. For example, gallium, copper, zinc, and silver possess known antimicrobial properties. Gallium is known to have anti-biofilm properties and is active against bacteria, planktonic cells, and biofilms because it competes with iron. It is believed that substitution of gallium for iron inactivates iron-containing enzymes necessary for bacterial growth. In other embodiments, other metals such as copper, zinc, or silver with known antimicrobial properties may be added to the formulation. In some embodiments, the antimicrobial agent contains two or more antimicrobial metals. In still other embodiments these metals are present in an oxide form or in organically available forms, such as silver oxide or silver taurate.

### E. Additional Ingredients

In some embodiments, additional ingredients known to those having skill in the art may be added to the formulation. These include, but are not limited to, those discussed below.

### 1. Antioxidants

Non-limiting examples of antioxidants that can be used with the antimicrobial agent of the present invention include acetyl cysteine, ascorbic acid polypeptide, ascorbyl dipalmitate, ascorbyl methylsilanol pectinate, ascorbyl palmitate, ascorbyl stearate, BHA, BHT, t-butyl hydroquinone, cysteine, cysteine HCI, diamylhydroquinone, di-t-butylhydroquinone, dicetyl thiodipropionate, dioleyl tocopheryl methylsilanol, disodium ascorbyl sulfate, distearyl thiodipropionate, ditridecyl thiodipropionate, dodecyl gallate, erythorbic acid, esters of ascorbic acid, ethyl ferulate, ferulic acid, gallic acid esters, hydroquinone, isooctyl thioglycolate, kojic acid, magnesium ascorbate, magnesium ascorbyl phosphate, methylsilanol ascorbate, natural botanical anti-oxidants such as green tea or grape seed extracts, nordihydroguaiaretic acid, octyl gallate, phenylthioglycolic acid, potassium ascorbyl tocopheryl phosphate, potassium sulfite, propyl gallate, quinones, rosmarinic acid, sodium ascorbate, sodium bisulfite, sodium erythorbate, sodium metabisulfite, sodium sulfite, superoxide dismutase, sodium thioglycolate, sorbityl furfural, thiodiglycol, thiodiglycolamide, thiodiglycolic acid, thioglycolic acid, thiolactic acid, thiosalicylic acid, tocophereth-5, tocophereth-10, tocophereth-12, tocophereth-18, tocophereth-50, tocopherol, tocophersolan, tocopheryl acetate, tocopheryl linoleate, tocopheryl nicotinate, tocopheryl succinate, and tris(nonylphenyl)phosphite.

### 2. Buffering systems

In some embodiments, the antimicrobial agent formulation further comprises a buffering system to maintain the formulation at an optimal pH. A suitable buffering system would be recognized by those having skill in the art, and include but are not limited to acetic acid/acetate, citric acid/citrate, glutamic acid/glutamate, and phosphoric acid/phosphate. The concentration of pH buffering system depends on the desired pH of the formulation. Any buffering system that is known to a person of skill in the art could be used with the antimicrobial agents. In a particular embodiment, the buffering system is Trizma HCl. In other embodiments, the buffer is a MES or HEPES buffer.

### 3. Surfactants

In some embodiments, the antimicrobial agent formulation further comprises a surfactant. The surfactant can be any suitable surfactant, which are well known to a person having skill in the art. Surfactants are wetting agents that lower the surface tension of a liquid to allow easier spreading and lower the interfacial tension between two liquids. In some embodiments of the present formulations, the surfactant is added to help to open up the biofilm to the antimicrobial properties of the formulation by emulsifying the mucopolysaccharides of the biofilm. The surfactant also aids the penetration of the formulation to address sub-surface biofilms. In particular embodiments, the surfactant is a mild surfactant. In other embodiments, the surfactant is a non-ionic surfactant.

### 4. Pharmaceutical Ingredients

Pharmaceutical ingredients are also contemplated as being useful with the antimicrobial agent formulations of the present invention. Non-limiting examples of pharmaceutical ingredients include anti-inflammatory agents including non-steroidal anti-inflammatory drugs, antibiotics, antifungals, antivirals, antimicrobials, anti-cancer actives, scabicides, pediculicides, antineoplastics, antiperspirants, antipruritics, antipsoriatic agents, antiseborrheic agents, biologically active proteins and peptides, burn treatment agents, cauterizing agents, depigmenting agents, depilatories, diaper rash treatment agents, enzymes, hemostatics, kerotolytics, canker sore treatment agents, cold sore treatment agents, dental and periodontal treatment agents, photosensitizing actives, skin protectant/barrier agents, steroids including hormones and corticosteroids, sunburn treatment agents, sunscreens, transdermal actives, and nasal actives. Other examples of pharmaceutical ingredients may further include deodorizers, antibiotics (such as erythromycin), antivirals, antiseptics (such as benzylthonium or benzylkonium chloride), and iron chelators that are antimicrobial, such as lactoferrin.

### F. Composition Vehicles

The compositions of the present invention can be formulated into all types of vehicles. Non-limiting examples of suitable vehicles include emulsions (*e.g*., oil-in-water, water-in-oil, silicone-in-water, water-in-silicone, water-in-oil-in-water, oil-in-water, oil-in-water-in-oil, oil-in-water-in-silicone, *etc*.), creams, lotions, solutions (both aqueous and hydro-alcoholic), anhydrous bases (such as lipsticks and powders), gels, ointments, pastes, milks, liquids, aerosols, solid forms, sprays, hydrogels, or electroporation device cartridges. In some embodiments, the formulation may be a hydrophilic solution, a thixotropic spray, or other hydrophillic topical. Variations and other appropriate vehicles will be apparent to the skilled artisan and are appropriate for use in the present invention. In certain aspects, the concentrations and combinations of the ingredients can be selected in such a way that the combinations are chemically compatible and do not form complexes which precipitate from the finished product. In still other aspects, the formulation may be immobilized on a surface, such as a dressing, and activated by a glucose wash.

### G. Methods of Use

In some embodiments, the invention provides for the use of an antimicrobial formulation such as those described above to treat or eliminate cells of a biofilm or planktonic biofilm-forming microorganisms. In such embodiments, the antimicrobial formulation may be applied to a surface or wound where biofilm exists or where planktonic biofilm-forming microorganism may be present and there is a high likelihood of a biofilm forming. The antimicrobial agent is contacted to the biofilm or potential biofilm to reduce or eliminate the cells of an existing biofilm or inhibit the growth of or eliminate cells of a biofilm-forming microorganisms.

Such compositions may be applied to a wound on a patient or applied to a surface, such as surgical instruments or infected hardware. For example, the antimicrobial agent as currently described may be used to solve problematic infections. Examples of such infections include, but are not limited to, common problems such as urinary tract infections, catheter infections, middle-ear infections, formation of dental plaque, gingivitis, coating contact lenses, endocarditis, infections in cystic fibrosis, infections associated with osteomyelitis, tinea corporis or tinea cruris, diaper rash, and nail fungus. Alternatively, the antimicrobial agent may be used as a coating for inert surfaces of implanted devices such as catheters, prosthetic cardiac valves, intrauterine devices, and tubes having entry sites to tissue. In some embodiments, the formulations may be used to facilitate cutaneous wound healing and increase topical antibacterial efficiency in healing or treating infected skin wounds. In other embodiments, it may be used in skin preps to protect periwound skin.

It has been reported that sub-inhibitory concentrations of antimicrobial agents may induce biofilm formation (*e.g.*, Frank *et al.,* 2007). In view of this, the lethal dosage for treatment of biofilm-forming microorganisms may be significantly higher than the standard therapeutically effective amount determined for planktonic microorganisms (*i.e*., a lethal amount or a lethal dosage) typically used by one of ordinary skill in the art. Thus, the standard therapeutically effective amount would be the amount of antimicrobial agent necessary to treat biofilm-forming microorganisms. A "standard therapeutic amount" or "standard therapeutic dose" may also refer to an amount of an agent sufficient to reduce or eliminate planktonic microorganisms. In some embodiments, treatment of biofilms and biofilm-forming microorganisms may require two or more doses of the antimicrobial agent.

### H. Combination treatments

The treatments of the present invention may be used on their own or in combination with additional methods of treatment. In order to increase the effectiveness of a treatment with the compositions of the present invention or to augment the protection of another (second) therapy, it may be desirable to combine these compositions and methods with other agents and methods effective in the treatment, reduction of risk, or prevention of infections, for example, anti-bacterial, anti-viral, and/or anti-fungal treatments. As another example, iontophoresis can be used to drive agents into tissues for the purpose of labeling or eradicating biofilms. Yet another example would be the use of the treatment with negative pressure wound therapy, such as V.A.C.@ Therapy (KCI International, San Antonio, TX). V.A.C.® Therapy delivers negative pressure at the wound site through a patented dressing to help draw wound edges together, remove infectious materials, and actively promote granulation at the cellular level. In a particular embodiment, Instillation Therapy is adapted to using separate reservoirs of enzyme and substrate solutions allows for the separation of substrate from the enzyme. The enzyme and substrate are only mixed at the tissue surface by introducing the two components in the delivery apparatus and mixing them with known static-mixer devices prior to introduction of the combined fluids into the wound space. This makes the formulation inherently more stable by storing the individual components of the formulation separately and preparing the whole formulation only at the site of use.

### I. Examples

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the scope of the invention.

### EXAMPLE 1

The objective of this study was to determine the ability of several antimicrobial preparations to inactivate *Pseudomonas aeruginosa* and *Staphylococcus aureus* biofilms on glass slides.

### 1. PROTOCOL OVERVIEW

*Pseudomonas aeruginosa* and *Staphylococcus aureus* were grown to form biofilms glass slides. Triplicate samples of each organism were exposed to one of three antimicrobial preparations (cocodimethylamine oxide, decyldimethylamine oxide, and lauramine oxide) for 10 minutes. After exposure, the remaining organism on each slide was assayed to determine the effects of each antimicrobial solution on each organism.

### 2. MATERIALS AND METHODS

### a. Preparation of the solutions

An experiment was conducted whereby three formulations were prepared according to the following example solution: 97.3998 % v/v diH2O; 0.6% w/v Tris HCl; 0.0002% (w/v) GaCl; 0.5% v/v zwitterionic detergent; 1.43% v/v Biovert® Enzyme Substrate; and 0.07% (v/v) Biovert® Enzyme. The zwitterionic detergent differed in the three solutions as shown:
- DO solution contained decylamine oxide
- LO solution contained lauramine oxide
- CDO contained cocamidopropylamine oxide.

All ingredients excepting the Biovert® substrate and enzyme were first mixed together. As a last step, the Biovert substrate and enzyme were added. The pH of the final solution should be between 5.2 - 5.9. In certain instances the pH may need to be adjusted using 1N NaOH.

### b. Preparation of microbial inocula

Fresh cultures of *Pseudomonas aeruginosa* (ATCC 09027) and *Staphylococcus aureus* (ATCC 29213) were revived from frozen stocks and streaked onto Tryptic Soy Agar (TSA, Becton Dickinson, Sparks, MD). TSA plates were incubated for 24 hours at 35°C. A single isolated colony of each culture was transferred into Tryptic Soy Broth (TSB, BD) and incubated for an additional 24 hours at 35°C. The biofilm formation protocol was taken from Harrison-Belestra *et al.* (2003) and is summarized as follows. Glass cover slips, cleaned with isopropyl alcohol, were suspended in a culture of each organism for 36-48 hours at 37°C and lightly agitated.

### c. Exposure to antimicrobial solutions and control

Triplicate prepared slides of each organism were exposed to each antimicrobial solution by spraying the slide with a predetermined volume of solution. Samples were exposed in sets of 3 slides 10 minutes. An additional set of prepared slides not exposed to an antimicrobial was prepared to determine the initial load of each organism.

### d. Enumeration of microorganisms

Total surviving microorganisms were enumerated as total colony-forming units per slide (CFU/slide). Individual slides were rinsed with sterile buffered peptone water (BPW, BD) and plated onto Pseudomonas Isolation Agar (PIA, BD) or Baird-Parker Agar (BP,BD) using an Eddy Jet spiral plater (IUL Instruments, Barcelona, Spain). Plates were incubated for 48 ± 2 hours at 35 ± 2°C and enumerated on an automated plate count reader (Flash and Go, IUL Instruments).

### 3. RESULTS

Results of the sample enumerations are shown in Tables 1 and 2 below, including the treatment applied to the slide, the observed amount of organism for each replicate, the average result of all three replicates, the log₁₀ of the average, and (for the antimicrobial treatments) the log reduction from the untreated control.

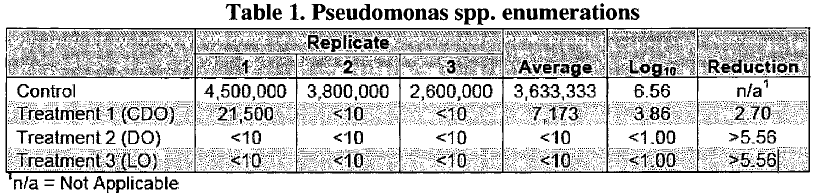

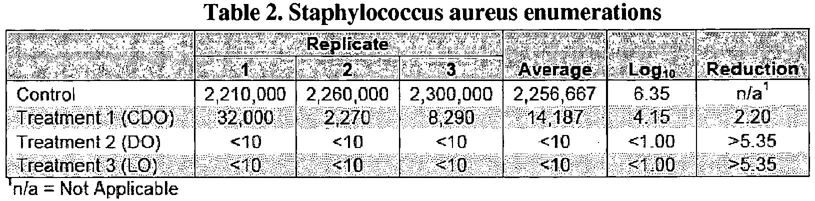

No organisms were recovered following exposure to either DO or LO solutions. This meant a minimum of a 5 log reduction for these two formulae. Exposure to CDO only led to a 2.70 log reduction of Pseudomonas and a 2.20 log reduction in Staphylococcus.

### 4. CONCLUSIONS

Decyldimethylamine oxide (DO) and lauramine oxide (LO) were effective at reducing more than 5 logs of *Pseudomonas aeruginosa* and *Staphylococcus aureus.* After 10 minutes of exposure with the antimicrobials, no organism was recoverable from the surface, a reduction of more than 5 logs from the untreated control samples. Cocodimethylamine oxide (CDO) was also able to reduce the test organisms, but at a lower efficacy (2.70 logs of reduction again *Pseudomonas* and 2.20 logs of reduction against *S. aureus*).

### EXAMPLE 2

Porcine skin explants are a recognized model for the study of biofilms *(see, e.g.,* Phillips et al., "Effects of Antimicrobial Agents on an In Vitro Biofilm Model of Skin Wounds," Advances in Wound Care, 1:299-304 (2010)). To determine the ability of antimicrobial preparations to inactivate biofilms in this model system, a *Pseudomonas* biofilm was grown on pig skin explants that were 5 inches wide by 7 inches long. Small wounds were made in the pig skin and the biofilm was developed in these wounds. All wounds in a particular pigskin were treated with the same agents. For example all wounds in one skin explant were treated with negative pressure wound therapy using V.A.C.@ Therapy, while all wounds in another were treated with Solution 1.

Explants were treated in the following manner: Wounds in the skin were covered with a polyurethane foam dressing having an average pore size of between 400-600 um. The dressings were covered with an occlusive drape (V.A.C.® GranuFoam™ Dressing). Two small holes were made in the drape. One was for application of a TRAC™ pad enabling the delivery of negative pressure (-125 mmHg) while the other was for a pad that allowed for the delivery of fluid to the explants. When delivering Solution 1 or Solution 2, the solutions were delivered to the skin explants 6 times a day with a solution dwell time on the skin of 10 minutes per instillation. When solution was being delivered to the skin, the V.A.C.@ Therapy unit was turned off. At the end of the 10 minute dwell period, the vacuum was turned back on which caused the fluid to be evacuated from the wounds in the skin explant. After 24 hours (and 6 solution instillation cycles) the bacteria remaining in the biofilm in the wounds was extracted and plated, grown and counted.

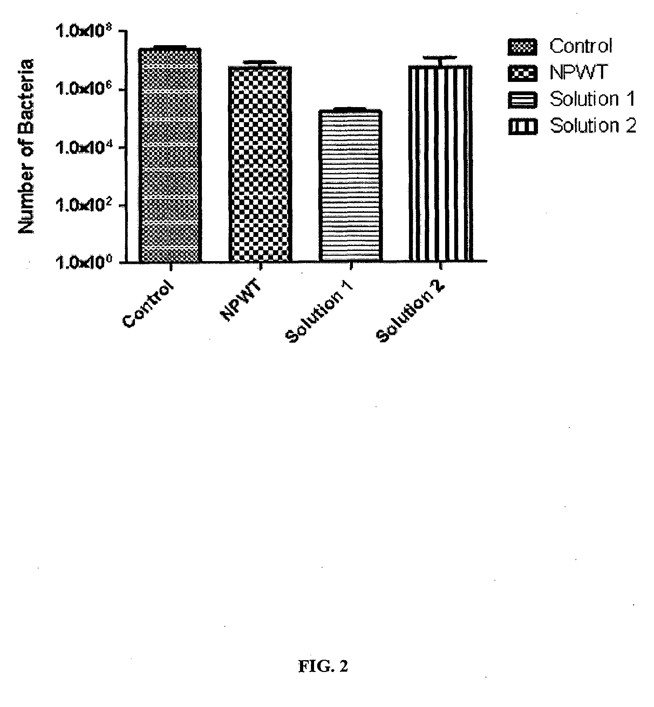

The conditions tested were:
Control - no treatment;
NPWT - treatment with V.A.C.® Therapy (only) at -125 mmHg and no solutions;
Solution 1 treatment - Lactoperoxidase, glucose oxidase, glucose, lauramine oxide, gallium chloride, Tris HCl; pH 5.3 - 5.9;
Solution 2 treatment - Lactoperoxidase, glucose oxidase, and glucose encapsulated in Spherulites™, Lauramine oxide, gallium chloride, Tris HCl, water; pH 5.2 - 5.9. Spherulites™ are multi-layered microspheres of surfactants that are used for the encapsulation of active ingredients. Spherulites™ can provide increased skin penetration and adhesion to biological surfaces, as well as time-release of active ingredients.
Solution 1 treatment led to an approximate 2 log reduction in bacteria (FIG. 2). After 6, 10 minute applications of the solution over the course of 24 hours, 93.2% of the *Pseudomonas* in the biofilm were killed. With Solution 2 77.1% of the *Pseudomonas* were killed. A ≥ 1 log reduction of bacteria is generally considered significant. Thus, the reduction achieved with Solution 1 treatment was significant, whereas the reduction achieved by Solution 2 was not necessarily significant. Furthermore, the application of negative pressure alone was not sufficient to cause a significant decrease in biofilm bacteria.

All of the compositions and/or methods disclosed and claimed herein can be made and executed without undue experimentation in light of the present disclosure. While the compositions and methods of this invention have been described in terms of some embodiments, it will be apparent to those of skill in the art that variations may be applied to the compositions and methods and in the steps or in the sequence of steps of the method described herein without departing from the concept and scope of the invention. More specifically, it will be apparent that certain agents which are both chemically and physiologically related may be substituted for the agents described herein while the same or similar results would be achieved. All such similar substitutes and modifications apparent to those skilled in the art are deemed to be within the scope and concept of the invention as defined by the appended claims.

### REFERENCES

Ananthapadmanabhan et al., Dermatologic Therapy, 17:16-25, 2004.
Frank et al. Antimicrobial Agents and Chemotherapy, 888-895, 2007.
Harrison-Belestra et al. Dermatol Surg., 29(6):631-635, 2003.
James et al., Wound Repair Regen., 16(1):37-44, 2008.
Kaneko et al., J. Clinical Invest., 117(4):877-888, 2007.
Michels et al., Soc. Applied Microbiol. Lett. Applied Microbiol., 49:191-195, 2009.
Phillips et al., Advances in Wound Care, 1:299-304, 2010.
Wolcott et al., J. Wound Care, 17(8):333-341, 2008.

## Claims

1. An antimicrobial composition comprising a lactoperoxidase, glucose oxidase, glucose, an antimicrobial metal gallium, and a zwitterionic detergent,
wherein the zwitterionic detergent is selected from the group consisting of decylamine oxidase and lauramine oxide; and
wherein the lactoperoxidase, glucose oxidase, and glucose collectively comprise about 1% to 5% (v/v) of the total composition.

2. The antimicrobial of claim 1, wherein the composition is formulated as an emulsion, spray, cream, lotion, ointment, or hydrogel.

3. The antimicrobial composition of claim 1 for use in the treatment of biofilm-forming microorganisms, the treatment comprising contacting the biofilm-forming microorganisms with the antimicrobial composition.

4. The composition for use according to claim 3, wherein the biofilm-forming microorganisms are in a planktonic state or are in a biofilm.

5. The composition for use according to claim 3, wherein the biofilm-forming microorganisms are located in a wound on a patient, optionally wherein the wound is selected from the group consisting of a burn, abrasion, cut, scrape, denuding tissue injury venous ulcer, diabetic ulcer, arterial ulcer, pressure ulcer, radiation ulcer, traumatic would, non-healing wound and combinations thereof.

6. The composition for use according to claim 5, wherein the antimicrobial composition is administered to the wound topically.

7. The composition for use according to claim 3, wherein the biofilm-forming microorganisms are Pseudomonas or Staphylococcus.

8. The composition for use according to claim 3, wherein the biofilm-forming microorganisms are contacted with the antimicrobial composition two or more times, optionally wherein the biofilm-forming microorganisms are contacted with the antimicrobial composition at least 6 times a day.

9. The composition for use according to claim 3, wherein the biofilm-forming microorganisms are located on a surgical instrument or an implanted device.

10. The composition for use according to claim 5, wherein the treatment further comprises applying negative pressure to the wound.

11. The composition for use according to claim 10, wherein the treatment further comprises covering the wound with a dressing prior to applying negative pressure, wherein the composition is applied to the dressing before applying the dressing to the wound on the patient.

## Patentansprüche

1. Antimikrobielle Zusammensetzung, die eine Lactoperoxidase, Glucoseoxidase, ein antimikrobielles Metallgallium und ein zwitterionisches Detergens umfasst,
wobei das zwitterionische Detergens ausgewählt ist aus der Gruppe, die aus Decylaminoxidase und Lauraminoxid besteht; und
wobei die Lactoperoxidase, Glucoseoxidase und Glucose zusammen etwa 1 % bis 5 % (v/v) der gesamten Zusammensetzung ausmachen.

2. Antimikrobielle Zusammensetzung nach Anspruch 1, wobei die Zusammensetzung als eine Emulsion, ein Spray, eine Creme, eine Lotion, eine Salbe oder als ein Hydrogel formuliert ist.

3. Antimikrobielle Zusammensetzung nach Anspruch 1 für eine Verwendung bei der Behandlung von Mikroorganismen, die einen Biofilm bilden, wobei die Behandlung ein Herstellen eines Kontaktes der Mikroorganismen, die einen Biofilm bilden, mit der antimikrobiellen Zusammensetzung umfasst.

4. Zusammensetzung für eine Verwendung nach Anspruch 3, wobei die Mikroorganismen, die einen Biofilm bilden, in einem planktonischen Zustand sind oder ein Biofilm sind.

5. Zusammensetzung für eine Verwendung nach Anspruch 3, wobei sich die Mikroorganismen, die einen Biofilm bilden, in einer Wunde eines Patienten befinden, wobei die Wunde wahlweise ausgewählt ist aus der Gruppe, die aus einer Verbrennung, einer Abschürfung, einer Schnittwunde, einer Kratzwunde, einer Verletzung einer Gewebeabtragung infolge eines Venengeschwürs, einem diabetischen Geschwür, einem arteriellen Geschwür, einem Druckgeschwür, einem Strahlungsgeschwür, einer traumatischen Wunde, einer nicht heilenden Wunde und aus Kombinationen davon besteht.

6. Zusammensetzung für eine Verwendung nach Anspruch 5, wobei die antimikrobielle Zusammensetzung auf die Wunde topisch verabreicht wird.

7. Zusammensetzung für eine Verwendung nach Anspruch 3, wobei die Mikroorganismen, die einen Biofilm bilden, Pseudomonas oder Staphylococcus sind.

8. Zusammensetzung für eine Verwendung nach Anspruch 3, wobei die Mikroorganismen, die einen Biofilm bilden, mit der antimikrobiellen Zusammensetzung zweimal oder mehrere Male in Kontakt miteinander gebracht werden, wobei die Mikroorganismen, die einen Biofilm bilden, mit der antimikrobiellen Zusammensetzung wahlweise mindestens 6mal pro Tag in Kontakt miteinander gebracht werden.

9. Zusammensetzung für eine Verwendung nach Anspruch 3, wobei sich die Mikroorganismen, die einen Biofilm bilden, auf einem chirurgischen Instrument oder auf einer implantierten Vorrichtung befinden.

10. Zusammensetzung für eine Verwendung nach Anspruch 5, wobei die Behandlung ferner die Ausübung eines Unterdrucks auf die Wunde umfasst.

11. Zusammensetzung für eine Verwendung nach Anspruch 10, wobei die Behandlung ferner ein Abdecken der Wunde mit einem Verband umfasst, bevor der Unterdruck ausgeübt wird, wobei die Zusammensetzung auf den Verband aufgetragen wird, bevor der Verband auf die Wunde des Patienten aufgetragen wird.

## Revendications

1. Composition antimicrobienne comprenant de la lactoperoxydase, de la glucose oxydase, du glucose, du gallium métal antimicrobien et un détergent zwitterionique,
dans laquelle le détergent zwitterionique est sélectionné à partir du groupe constitué par la décylamine oxydase et l'oxyde de lauryldiméthylamine ; et
dans laquelle la lactoperoxydase, la glucose oxydase et le glucose comprennent collectivement environ 1 % à 5 % (V/V) de la composition totale.

2. Produit antimicrobien selon la revendication 1, dans lequel la composition est formulée comme une émulsion, une vaporisation, une crème, une lotion, un onguent, ou un hydrogel.

3. Composition antimicrobienne selon la revendication 1, à utiliser dans le traitement de micro-organismes formant un film biologique, le traitement comprenant la mise en contact des micro-organismes formant un film biologique avec la composition antimicrobienne.

4. Composition à utiliser selon la revendication 3, dans laquelle les micro-organismes formant un film biologique sont dans un état planctonique ou sont dans un film biologique.

5. Composition à utiliser selon la revendication 3, dans laquelle les micro-organismes formant un film biologique sont placés dans une blessure chez un patient, de manière facultative dans laquelle la blessure est sélectionnée à partir du groupe constitué par une brûlure, une écorchure, une coupure, une égratignure, un ulcère veineux à lésion tissulaire mise à nue, un ulcère diabétique, un ulcère artériel, une escarre de décubitus, un ulcère roentgenien, une blessure traumatique ne cicatrisant pas, et des combinaisons de ceux-ci.

6. Composition à utiliser selon la revendication 5, dans laquelle la composition antimicrobienne est administrée à la blessure par voie topique.

7. Composition à utiliser selon la revendication 3, dans laquelle les micro-organismes formant un film biologique sont *Pseudomonas* ou *Staphylococcus.*

8. Composition à utiliser selon la revendication 3, dans laquelle les micro-organismes formant un film biologique sont mis en contact avec la composition antimicrobienne deux fois ou plus, de manière facultative dans laquelle les micro-organismes formant un film biologique sont mis en contact avec la composition antimicrobienne au moins 6 fois par jour.

9. Composition à utiliser selon la revendication 3, dans laquelle les micro-organismes formant un film biologique sont placés sur un instrument chirurgical ou un dispositif implanté.

10. Composition à utiliser selon la revendication 5, dans laquelle le traitement comprend en outre l'application d'une pression négative à la blessure.

11. Composition à utiliser selon la revendication 10, dans laquelle le traitement comprend en outre le recouvrement de la blessure par un pansement avant d'appliquer une pression négative, dans laquelle la composition est appliquée au pansement avant l'application du pansement à la blessure sur le patient.
